(19) Europäisches Patentamt
European Patent Office
Office européen des brevets

(11) Numéro de publication: **0 377 539 A2**

# (12) DEMANDE DE BREVET EUROPEEN

(21) Numéro de dépôt: **90400041.1**

(51) Int. Cl.5: **A61K 31/40**

(22) Date de dépôt: **05.01.90**

Revendications pour les Etats contractants suivants: ES + GR.

(30) Priorité: **06.01.89 FR 8900117**

(43) Date de publication de la demande: **11.07.90 Bulletin 90/28**

(84) Etats contractants désignés: **AT BE CH DE DK ES FR GB GR IT LI LU NL SE**

(71) Demandeur: **Albert ROLLAND S.A. Société dite**
**49, Rue St-André-des-Arts**
**F-75006 Paris(FR)**

(72) Inventeur: **Bessin, Pierre**
**1 Alleé Bossuet**
**F-91380 Chilly Mazarin(FR)**

(74) Mandataire: **Burtin, Jean-François**
**Bugnion Associés Département GEF1B 55 Rue Boissonade**
**F-75014 Paris(FR)**

## (54) Nouvelles compositions pharmaceutiques manifestant des propriétés inhibitrices vis à vis de la phospholipase A2.

(57) La présente invention se rapporte à de nouvelles compositions pharmaceutiques manifestant des propriétés inhibitrices vis-à-vis de la phospholipase $A_2$.

Elle renferme à titre de principe actif au moins un dérivé d'acide (4-aroyl pyrrolyl-2) carboxylique de formule générale I

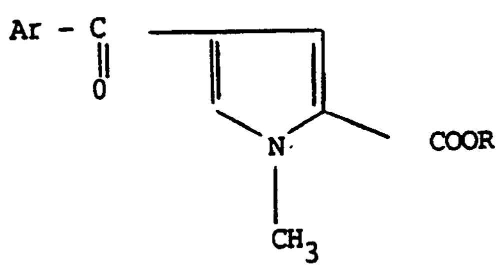

(I)

L'invention concerne également l'utilisation des composés de formule générale I en vue de la réalisation d'un médicament assurant l'inhibition in vivo des phospholipases $A_2$.

EP 0 377 539 A2

Xerox Copy Centre

# NOUVELLES COMPOSITIONS PHARMACEUTIQUES MANIFESTANT DES PROPRIETES INHIBITRICES VIS-À-VIS DE LA PHOSPHOLIPASE $A_2$.

La présente invention se rapporte à des dérivés de l'acide N-méthyl pyrrolyl-2 carboxylique.

Elle a plus particulièrement pour objet une nouvelle application thérapeutique d'acides aroyl N-méthyl pyrrolyl-2 carboxyliques.

L'invention a spécifiquement pour objet des compositions pharmaceutiques manifestant des propriétés inhibitrices vis-à-vis de la phospholipase $A_2$ renferment à titre de principe actif au moins un dérivé (4-aroyl pyrrolyl-2) carboxylique de formule générale I

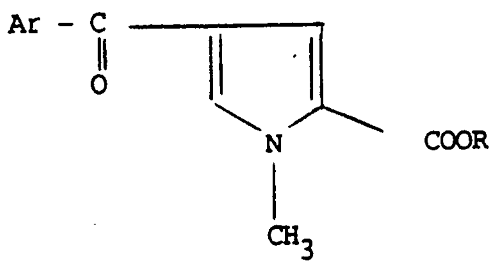

(I)

dans laquelle Ar est choisi dans le groupe constitué par un phényle, un halogénophényle, un naphtyl-1 et un naphtyl-2 et R est de l'hydrogène ou le cation monovalent d'une base minérale ou organique thérapeutiquement compatible

en association ou en mélange avec un excipient ou un véhicule inerte, non toxique, pharmaceutiquement acceptable.

Les acides pyrrolyl-2 carboxyliques principes actifs utilisés dans la présente invention, sont des produits connus. Certains ont déjà été déccrits dans le brevet US 2.479.972 en tant qu'anesthésiques locaux et surtout dans le brevet français 2.405.246 au nom de la demanderesse.

L'étude pharmacologique de ces composés avait montré qu'ils possédaient une activité uricosurique et pouvaient de ce fait, être utilisés dans le traitement des hyperuricémies pathologiques comme dans la goutte ou pour éviter une augmentation de l'acide urique sanguin à la suite de la prise de certains médicaments.

Par la suite, dans son certificat d'utilité 2.564.831, la demanderesse a montré que les composés de formule générale I manifestaient également des propriétés immuno-suppressives qui se concrétisent notamment par une inhibition du phénomène de rosettes. De ce fait, ces composés sont utiles pour le traitement des maladies auto-immunes, pour la prévention du rejet des greffes, dans la prévention et le traitement des réactions de rejet du greffon vis-à-vis de l'hôte.

Or, il a été trouvé maintenant et c'est sur cette connaissance que repose la présente invention, que les composés de formule générale I possèdent également une activité antiphospholipase $A_2$ à la fois in vitro vis-à-vis d'une phospholipase $A_2$ de pancréas de porc et vis-à-vis des phospholipases $A_2$ cellulaires et principalement une phospholipase de monocytes humains.

Cette propriété dans l'état actuel des connaissances de la demanderesse était inattendue et surtout ne pouvait découler des propriétés pharmacologiques déjà connues de ces composés.

Dans ce qui précède, les sels des composés de formule I avec une base minérale ou organique peuvent être définis comme des sels de métaux alcalins, comme le sodium, le potassium, le lithium, l'ammonium ou le rubidium ; des sels de métaux alcalion-terreux comme le calcium ou le strontium ; le magnésium, l'aluminium ou les métaux ferreux. Ont peut également y inclure les sels d'alcoylamines, de cyclo-alcoylamines, d'aryl-alcoyl-amines, de pyridyl-alcoylamines, de furyl-alcoylamines, d'hydroxyl-alcoylamines, d'amino acides, de desoxy-osamines, d'amino cyclitols, de guanidines substituées, de poly peptides ou de composés similaires.

Les compositions pharmaceutiques selon l'invention sont utiles pour le traitement ou la prévention de phénomènes inflammatoires ou douloureux et principalement pour le traitement précoce des maladies dégénératives ostéo-articulaires comme le rhumatisme articulaire, la spondylarthrite ankylosantes ou la polyarthrite rhumatoïde.

En effet, l'inhibition des phospholipases $A_2$ empêche la mise en liberté de l'acide arachidonique et par voie de conséquence, supprime la formation de ce qu'on appelle la cascade des métabolites de l'Acide

Arachidonique, c'est-à-dire les prostaglandines, les leucotriènes et les lipoxines.

Ces composés qui sont des médiateurs de l'inflammation sont responsables du démarrage des phénomènes douloureux d'abord puis des phénomènes de bloquage des articulations.

L'action des composés de formule I est donc différente de celles des glucocorticoïdes dont l'action inhibitrice des phospholipases $A_2$ s'effectue par induction de la synthèse d'une protéine qui se lie à et bloque la phospholipase $A_2$ (cf. demande de brevet européen 213.916). On dispose donc d'un médicament nouveau qui agit à un stade précoce sur les phénomènes inflammatoires et sur les perturbations cellulaires graves qu'ils entrainent.

La posologie utile peut varier dans de larges proportions en fonction de la nature et de l'ancienneté de la maladie rhumatoïde. En règle générale, elle varie de 25 à 500 mg de principe actif de formule générale I par prise unitaire et de préférence de 50 à 250 mg. La posologie journalière s'échelonne de 50 mg à 1000 mg en fonction du poids du sujet et de la sévérité de la pathologie à traiter.

Les compositions pharmaceutiques selon l'invention sont destinées à être administrées par voie parentérale, orale, rectale, per muqueuse ou per cutanée.

A cette fin, les compositions pharmaceutiques se présentent sous la forme de comprimés nus ou enrobés, de dragées, de gélules, de capsules, de gouttes, de solutions ou suspensions buvables, de solutés ou suspensions injectables, de suppositoires ou de solutions dans un solvant polaire pour l'usage per cutané.

Les exemples suivants illustrent l'invention sans toutefois la limiter.

| **EXEMPLE I** | |
|---|---|
| Comprimés à 0,250 g d'acide 4-[(Naphtyl-2) carbonyl] N-méthyl pyrrolyl-2 carboxylique | |
| Acide 4-[(Naphtyl.2) carbonyl] N-méthyl pyrrolyl-2 carboxylique..... | 250 g |
| Amidon de blé..... | 125 g |
| Amidon de Maïs..... | 62 g |
| Silice colloïdale..... | 25 g |
| Ethylcellulose..... | 20 g |
| Hydroxy propyl méthylcellulose..... | 23 g |
| Talc..... | 15 g |
| pour 1.000 comprimés terminés au poids moyen de | 0,500 g |

**EXEMPLE II**

Etude pharmacologique des composés de formule générale I

Le but du travail était de rechercher si les composés de formule I étaient capables d'inhiber la phospholipase $A_2$ de pancréas de porc "in vitro", et des phospholipases $A_2$ cellulaires, principalement une phospholipase $A_2$ de monocytes humains.

Deux tests ont été utilisés :

1- Un test "in vitro", utilisant comme substrat, des membranes d'E.Coli marquées en position 2 par de l'acide oléique tritié, et comme enzyme, une phospholipase $A_2$ de pancréas de porc.

2- Un test sur cellules entières, marquées à l'acide arachidonique tritié, et dont l'activité phospholipasique $A_2$ est stimulée par du ionophore A23187.

Le composé de formule I testé, a été incubé, dans ces systèmes, à différentes concentrations, avec l'enzyme au préalable, dans le premier cas, et avec les cellules, dans le deuxième cas.

Les résultats suivants ont été obtenus avec l'acide 4-(Naphtyl-2 carbonyl) N-méthyl pyrrolyl-2 carboxylique.

1- RESULTATS OBTENUS DANS LE TEST "IN VITRO" (PHOSPHOLIPASE A2 MEMBRANAIRE

Après incubation de 10 minutes, l'acide 4-(Naphtyl-2 carbonyl) N-méthyl pyrrolyl-2 carboxylique s'est montré inhibiteur de la phospholipase $A_2$ de porc, au niveau membranaire, avec une IC50 de 2.10-4M.

Dans des essais impliquant plusieurs concentrations de substrat, il a été démontré, par ailleurs, que l'inhibition de la phospholipase $A_2$ était indépendante de la dose de substrat, donc qu'il s'agissait d'une inhibition de l'enzyme et non pas de liaison de la drogue au substrat.

2- RESULTATS OBTENUS SUR MONOCYTES HUMAINS STIMULES PAR LE IONOPHORE A23187 (PHOSPHOLIPASE A2 CELLULAIRE)

Les essais réalisés sur monocytes humains, stimulés par le ionophore A23187, ont montré que l'acide 4-(Naphtyl-2 carbonyl) N-méthyl pyrrolyl-2 carboxylique avait également une activité antiphospholipase $A_2$ cellulaire, avec une IC50 de 2.10-4M.

En conclusion, ces essais ont démontré que les composés de formule I, et principalement l'acide 4-(Naphtyl-2 carbonyl) N-méthyl pyrrolyl-2 carboxylique, avaient une activité antiphospholipase $A_2$ in vitro membranaire et cellulaire. Dans l'état actuel des recherches, l'activité antiphospholipase $A_2$ des composés de l'invention procède directement de l'inhibition de l'enzyme, se différenciant donc des corticoïdes qui agissent indirectement par l'intermédiaire de la synthèse de protéines inhibitrices.

**INHIBITION DE LA PHOSPHOLIPASE A2 par**
**l'ACIDE 4-(NAPHTYL-2 CARBONYL) N-MÉTHYL PYRROLYL-2 CARBOXYLIQUE**

ETUDE IN VITRO

1) **PHOSPHOLIPASE A2 MEMBRANAIRE : MEMBRANE d'E.COLI**

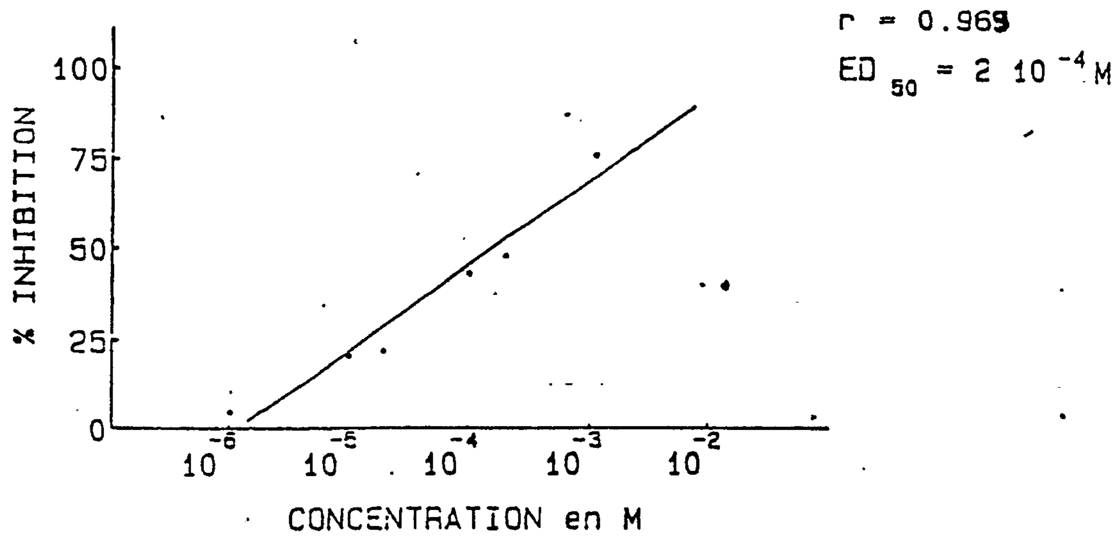


2) **PHOSPHOLIPASE A2 CELLULAIRE : MONOCYTES HUMAINS**

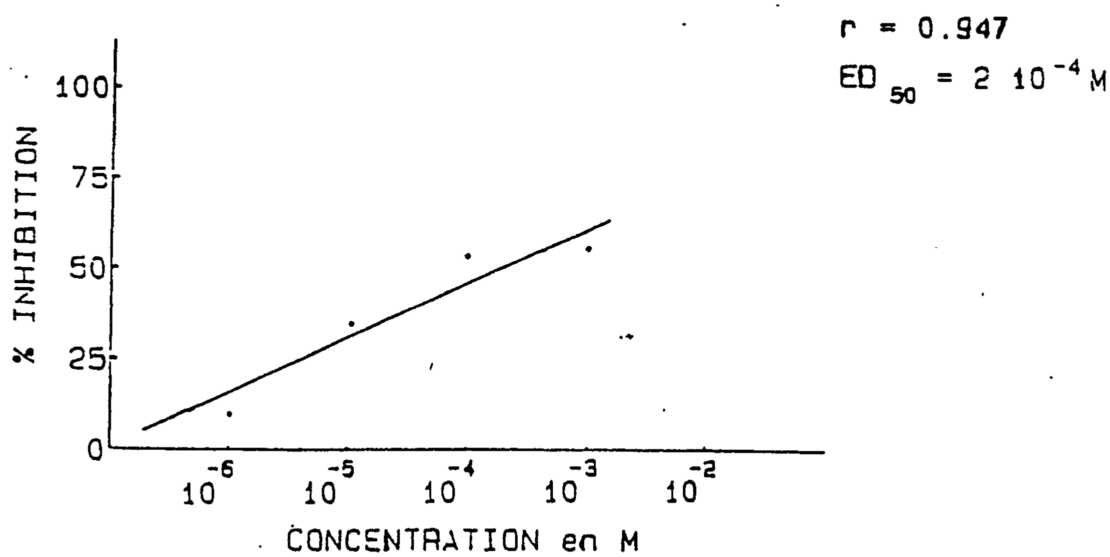


## Revendications

1°- Utilisation des composés de formule générale I

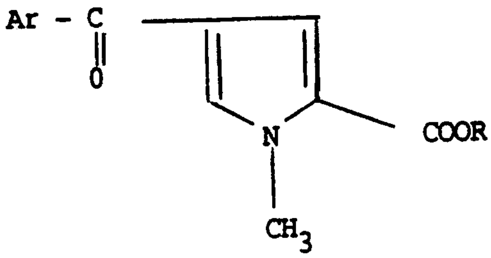

(I)

dans laquelle Ar est choisi dans le groupe constitué par un phényle, un halogénophényle, un naphtyl-1 et un naphtyl-2 et R est de l'hydrogène ou le cation monovalent d'une base minérale ou organique thérapeutiquement compatible
en association ou en mélange avec un excipient ou un véhicule inerte, non toxique, pharmaceutiquement acceptable.
en vue de la réalisation d'un médicament assurant l'inhibition "in vivo", des phospholipases $A_2$.

2°- Utilisation selon la revendication 1° d'un composé de formule générale 1 à savoir l'acide 4-(Naphtyl-2 carbonyl) N-méthyl pyrrolyl-2 carboxylique en vue de la réalisation d'un médicament assurant l'inhibition "in vivo" des phospholipases $A_2$

3°- Utilisation selon la revendication 1° ou la revendication 2° d'un composé de formlule générale I en vue de la réalisation d'un médicament assurant l'inhibition "in vivo", des phospholipases $A_2$ à savoir un sel de métal alcalin de l'acide 4-(Naphtyl-2 carbonyl) N-méthyl pyrrolyl-2 carboxylique.

4°- Utilisation selon l'une des revendications 1 à 3° d'un composé de formule générale I en vue de la réalisation d'un médicament assurant l'inhibition "in vivo", des phospholipases $A_2$ caractérisée en ce que le médicament est un de ceux qui conviennent pour l'administration par voie parentérale, orale, rectale, permuqueuse ou percutanée.

5°- Utilisation selon l'une des revendications 1 à 4° d'un composé de formule générale I dans laquelle, la teneur en principe actif dans le médicament assurant l'inhibition "in vivo" des phospholipases $A_2$ s'échelonne de 25 à 500 mg par prise unitaire.

6°- Utilisation selon l'une des revendications 1 à 5° d'une composé de formule générale I dans laquelle la teneur en principe actif de formule générale I s'échelonne de 50 à 250 mg par prise unitaire.

Revendications pour les Etats contractants suivants: ES, GR

1°- Procédé pour la réalisation d'un médicament assurant l'inhibition in vivo des phospholipases $A_2$ caractérisé en ce qu'un composé de formule générale I

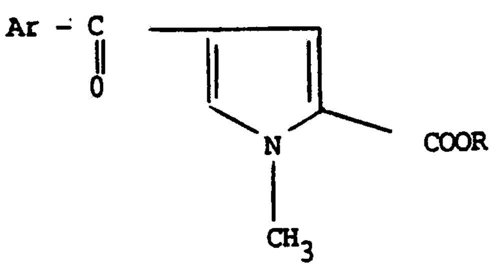

(I)

dans laquelle Ar est choisi dans le groupe constitué par un phényle, un halogénophényle, un naphtyl-1 et un naphtyl-2 et R est de l'hydrogène ou le cation monovalent d'une base minérale ou organique thérapeutiquement compatible.
est associé ou mélangé avec un excipient ou un véhicule inerte non toxique pharmaceutiquement acceptable.

2°- Un procédé selon la revendication 1° dans lequel le principe actif de formule générale I est l'acide 4-(naphtyl-2 carbonyl) N-méthyl pyrrolyl-2 carboxylique.

3°- Un procédé selon la revendication 1° dans lequel le principe actif de formule générale I est un sel de métal alcalin de l'acide 4-(Naphtyl-2 carbonyl) N-methyl pyrrolyl-2 carboxylique.

4°- Un procédé selon la revendication 1° dans lequel la quantité de principe actif dans le médicament

assurant l'inhibition in vivo des phospholipases $A_2$ s'échelonne de 25 à 500 mg par prise unitaire.